# FASCICULE DE BREVET EUROPEEN

(11) **EP 3 678 788 B1**
(45) Date de publication et mention de la délivrance du brevet: **10.11.2021**
(21) Numéro de dépôt: 18773936.2
(22) Date de dépôt: 07.09.2018
(51) Int. Cl.: B05B 7/10, B05B 1/34, B05B 7/24, A61L 2/22

(54) **BUSE DE NEBULISATION POUR APPAREIL DE DESINFECTION DE SURFACE PAR VOIE AERIENNE**
SPRÜHDÜSE FÜR EINE VORRICHTUNG ZUR DESINFEKTION VON OBERFLÄCHEN AUS DER LUFT
SPRAY NOZZLE FOR AN APPARATUS FOR AERIALLY DISINFECTING SURFACES

(30) Priorité: 08.09.2017 FR 1770945
(43) Date de publication de la demande: 15.07.2020
(73) Titulaire: Airinspace, 78990 Elancourt (FR)
(72) Inventeur: DE LINAGE, Pierre, 92100 Boulogne-Billanccourt (FR); KLACZYNSKI, Nicolas, 78760 Jouars-Pontchartrain (FR)
(74) Mandataire: Barbot, Willy
(86) Numéro de dépôt international: PCT/EP2018/000429
(87) Numéro de publication internationale: WO 2019/048079

(56) Documents cités:
- GB-A- 827 309
- GB-A- 859 114
- US-A- 6 076 744
- US-A1- 2004 098 989

## Description

La présente demande de brevet revendique la priorité de la demande de brevet français FR 17/70945 déposée en date du 8 septembre 2017.

La présente invention concerne le domaine de la désinfection d'un environnement par voie aérienne. Il est connu dans ce domaine de nébuliser un produit de traitement, tel un produit biocide, en le mélangeant avec un air propulseur afin de le diffuser en fines gouttelettes dans l'environnement, afin de traiter les surfaces de ce dernier.

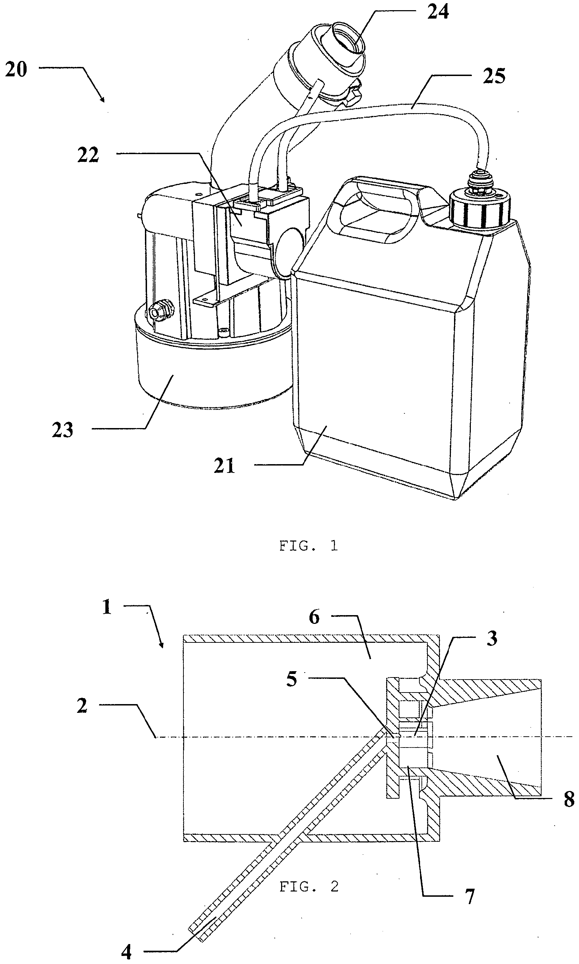

Il est connu pour cela d'utiliser un appareil 20, tel que celui illustré à la figure 1, comprenant un réservoir 21 de produit de traitement, un moyen de transport, comprenant par exemple une pompe 22, et une canalisation 25 (ou 4) de ce produit jusqu'à une buse 24 (ou 1) et un moto-ventilateur 23 apte à souffler l'air jusqu'à ladite buse 24. La fonction de la buse 24 est de nébuliser le produit de traitement, de le mélanger avec l'air propulseur et de permettre la diffusion du mélange air/produit vers l'environnement à traiter.

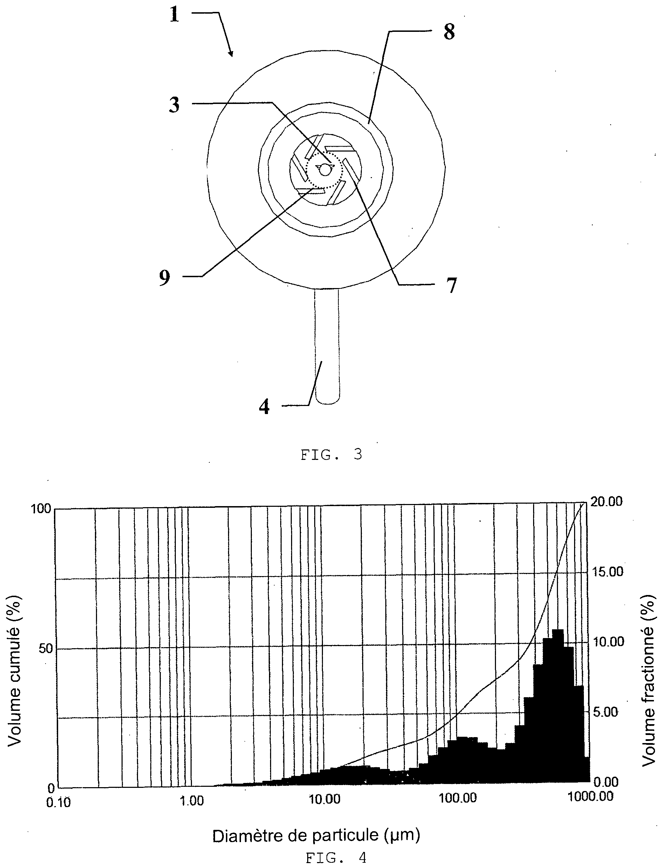

Telle qu'illustrée aux figures 2 et 3, une telle buse de nébulisation 1 est une pièce sensiblement de révolution autour d'un axe 2, comprenant une chambre 3 sensiblement cylindrique d'axe confondu avec l'axe 2. Le produit de traitement arrive dans la buse 1 par une canalisation 4. Cette canalisation débouche dans le fond 5 de la chambre 3, préférentiellement au centre dudit fond 5. La buse 1 comprend encore une entrée d'air 6. Cette entrée d'air 6 reçoit l'air en provenance du moto-ventilateur. Elle est circulaire autour de la périphérie de la chambre 3 afin de permettre à l'air de pénétrer dans la chambre 3. Des ailettes 7 sont disposées entre l'entrée d'air 6 et la chambre 3 afin de guider le flux d'air. La buse 1 comprend encore un diffuseur 8 sensiblement conique s'ouvrant vers l'extérieur, d'axe aligné avec l'axe 2 de la buse 1. Ce diffuseur 8 est accolé à la chambre 3 du côté opposé au fond 5. Selon l'art antérieur, tel que plus particulièrement illustré aux figures 2 et 3, les ailettes 7 sont planes et sensiblement radiales relativement à l'axe 2.

Une telle buse 1 permet de nébuliser le produit de traitement et de le diffuser dans un environnement. Cependant une analyse granulométrique du flux sortant d'une telle buse 1 montre un spectre de taille de gouttelettes, tel qu'illustré à la figure 4, très large et très irrégulier, ce qui est préjudiciable en termes d'efficacité de la désinfection.

Document GB859114 décrit un autre exemple de buse pour nébuliser un liquide.

Aussi il convient d'améliorer l'appareil 20 et/ou la buse 1 afin d'homogénéiser la taille des gouttelettes, réduire le spectre de taille des gouttelettes, afin d'améliorer l'efficacité de la désinfection.

La présente invention remédie à ces différents inconvénients et propose, selon un premier objet, une buse de nébulisation pour un appareil de désinfection de surface par voie aérienne, sensiblement de révolution autour d'un axe, comprenant une chambre sensiblement cylindrique d'axe confondu avec l'axe de la buse, une canalisation débouchant dans le fond de la chambre, préférentiellement au centre dudit fond, apte à apporter un produit de traitement, une entrée d'air circonvenant la périphérie de la chambre, des ailettes disposées entre l'entrée d'air et la chambre et un diffuseur sensiblement conique s'ouvrant vers l'extérieur, d'axe aligné avec l'axe de la buse et accolé à la chambre du côté opposé au fond, où les ailettes sont courbes.

Selon une autre caractéristique, les ailettes sont parallèles à l'axe de la buse et équi-angulairement réparties autour de l'axe de la buse.

Selon une autre caractéristique, la concavité de la courbure des ailettes est dirigée vers l'intérieur de la buse.

Selon une autre caractéristique, les ailettes présentent une forme de spirale initiée sur un cercle centré sur l'axe de la buse.

Selon une autre caractéristique, la spirale est de type logarithmique.

Selon une autre caractéristique, le nombre d'ailettes est compris entre 3 et 10, et préférentiellement égal à 5.

Selon une autre caractéristique, le diffuseur présente un congé arrondi sur son arrête interne de sortie.

Selon une autre caractéristique, le diffuseur comprend, en s'éloignant de la chambre, un premier cône, un deuxième cône de plus grand diamètre et un décrochement entre les deux cônes.

Selon une autre caractéristique, le décrochement présente un congé arrondi.

Un deuxième objet porte sur un appareil de désinfection de surface par voie aérienne comprenant un réservoir de produit de traitement, un moyen de transport, comprenant par exemple une pompe, et une canalisation de ce produit jusqu'à une buse et un moto-ventilateur apte à souffler l'air jusqu'à ladite buse, laquelle buse est telle que décrite précédemment et laquelle buse est comprise dans cet appareil de désinfection.

Un troisième et dernier objet de l'invention porte sur une utilisation d'une buse telle que décrite précédemment pour la désinfection de surface par la nébulisation d'un produit de traitement.

D'autres caractéristiques, détails et avantages de l'invention ressortiront plus clairement de la description détaillée donnée ci-après à titre indicatif en relation avec des dessins sur lesquels :
- la figure 1, déjà décrite, présente un appareil désinfection de surface par voie aérienne,
- les figures 2 et 3, déjà décrites, présentent une buse selon l'art antérieur, respectivement en vue coupée de profil et en vue de face,
- la figure 4, déjà décrite, présente l'analyse granulométrique du jet obtenu au moyen de la buse des figures 2 et 3,
- les figures 5 et 6 présentent une buse selon un premier mode de réalisation de l'invention, respectivement en vue coupée de profil et en vue de face,
- la figure 7 présente l'analyse granulométrique du jet obtenu au moyen de la buse des figures 5 et 6,
- les figures 8 et 9 présentent une buse selon un autre mode de réalisation de l'invention, respectivement en vue coupée de profil et en vue de face,
- les figures 10 et 11 présentent une buse selon encore un autre mode de réalisation de l'invention, respectivement en vue coupée de profil et en vue de face.

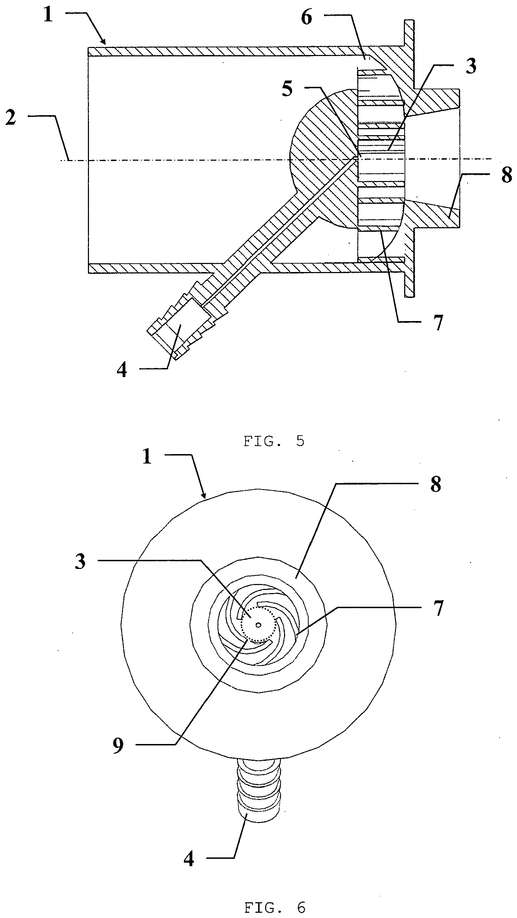

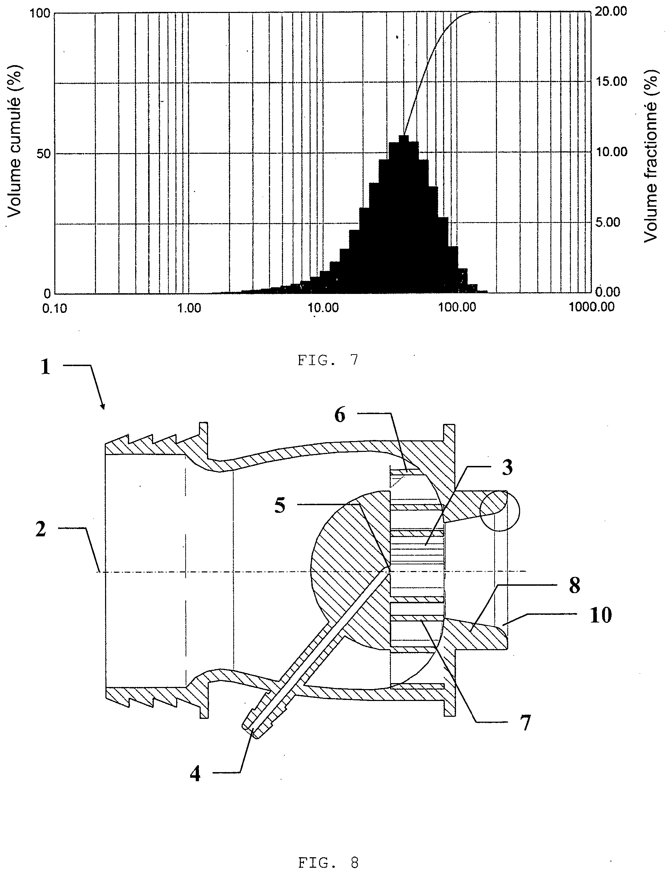

Selon une première caractéristique de l'invention, plus particulièrement illustrée aux figures 5 et 6, une buse est en tous points identique à une buse de l'art antérieur telle qu'illustrée aux figures 2 et 3. Elle se distingue et se caractérise cependant en ce que les ailettes 7 sont courbes. Cette courbure permet avantageusement de mettre l'air entrant dans la chambre 3 en mouvement circulaire selon un vortex. Ce vortex a pour conséquence une meilleure nébulisation du produit de traitement qui conduit à une meilleure homogénéité des gouttelettes avec un spectre moins étendu. Ceci est particulièrement visible à la figure 7 qui illustre l'analyse granulométrique du jet obtenu au moyen de la buse des figures 5 et 6 comportant des ailettes 7 courbes. L'analyse granulométrique est présentée sous forme d'un spectre figurant le diamètre des gouttelettes en abscisse, exprimé en microns, et la fréquence en ordonnée, exprimée en pourcentage, pour le graphique à barres, et le volume cumulé en ordonnée, exprimé en pourcentage, pour la courbe continue. Il peut ainsi être observé, en comparant le spectre de la figure 4, correspondant à une buse selon l'art antérieur, au spectre présenté à la figure 7, correspondant à une buse selon l'invention, que la distribution des gouttelettes présente une meilleure régularité, avec un spectre sensiblement gaussien et surtout présentant une étendue avantageusement beaucoup plus réduite. Un tel spectre d'étendue restreinte permet d'améliorer la maîtrise du cône de diffusion. La distribution des gouttelettes présente encore avantageusement un diamètre moyen beaucoup plus petit, ce qui favorise encore la nébulisation.

Selon une autre caractéristique, les ailettes 7 sont parallèles à l'axe 2 de la buse 1. Elles sont encore avantageusement équi-angulairement réparties autour de l'axe 2 de la buse 1.

Afin d'optimiser l'effet de vortex obtenu du fait de la courbure des ailettes 7, cette courbure est la même pour toutes les ailettes 7 et est telle que sa concavité fasse face à l'intérieur de la buse 1.

Selon un mode de réalisation préféré la courbe des ailettes 7 présente une forme de spirale. Afin de laisser une partie de la chambre 3 libre, les ailettes 7 débutent sur un cercle 9 centré sur l'axe 2 de la buse 1.

Selon un mode de réalisation encore préféré, ladite spirale est de type logarithmique.

Le nombre d'ailettes peut être quelconque. Selon un mode de réalisation préféré, il est compris entre 3 et 10. Selon un mode de réalisation encore préféré il est égal à 5.

Il a été vu que le diffuseur 8 présentait une forme conique. Avec une arrête interne de sortie du diffuseur 8 standard, il est constaté sur l'extrémité du diffuseur 8 une accumulation de gouttelettes de taille importante. Ces gouttelettes n'apparaissent pas sur le spectre car elles sont stoppées avant de croiser l'instrument de mesure. Cependant ce phénomène est préjudiciable en ce qu'il constitue un gaspillage de produit de traitement non véritablement utile, puisque non diffusé.

Afin de supprimer cet effet préjudiciable, le diffuseur présente un congé 10 sur son arrête interne de sortie. Ce congé 10 est préférentiellement arrondi, avec une concavité faisant face à l'extérieur du cône.

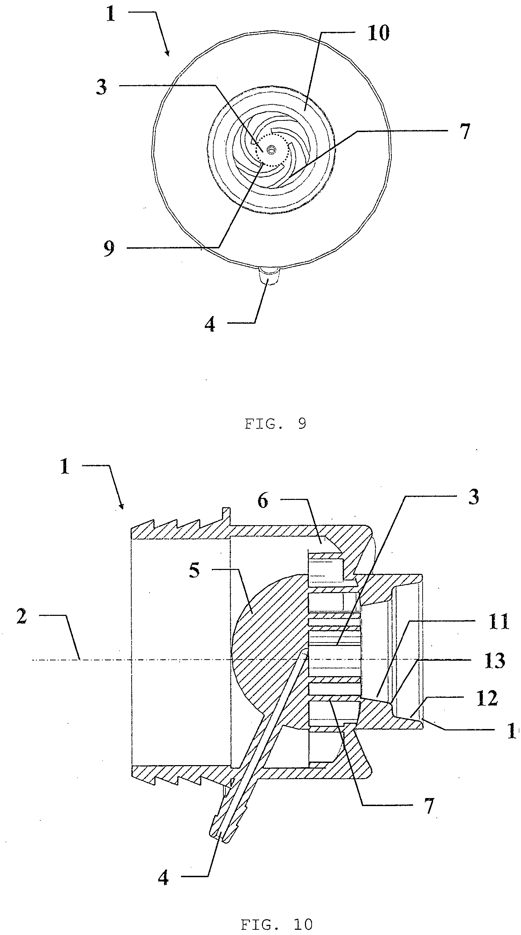

Une telle modification du diffuseur 8 ne modifie sensiblement pas le spectre de granulométrie. La buse illustrée aux figures 8 et 9 conserve ainsi les avantages procurés par l'invention.

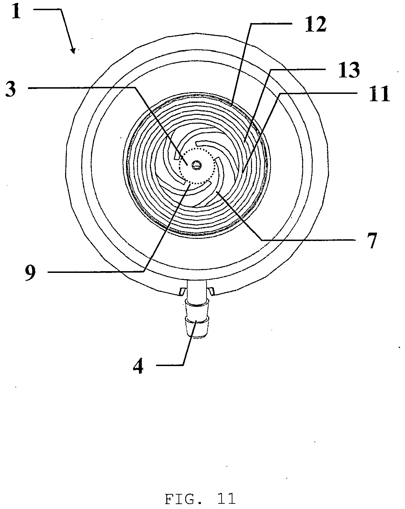

Selon une autre caractéristique, plus particulièrement illustrée aux figures 10 et 11, le diffuseur 8 comprend, en s'éloignant de la chambre 2, un premier cône 11, un deuxième cône 12 de plus grand diamètre et un décrochement 13 entre les deux cônes 11, 12.

Une telle disposition permet avantageusement de raccourcir la longueur de cône, réduite à la longueur du premier cône 11, potentiellement en contact avec le flux diffusé et permet ainsi de réduire les frottements entre le mélange diffusé et le diffuseur 8. Le deuxième cône 12 contribue, avantageusement sans contact, à protéger le flux diffusé.

Le décrochement 13 présente avantageusement, pour les mêmes raisons que précédemment, un congé arrondi, similaire au congé 10.

Une telle modification du diffuseur 8 ne modifie sensiblement pas le spectre de granulométrie. La buse illustrée aux figures 10 et 11 conserve ainsi les avantages procurés par l'invention.

## Revendications

1. Une buse de nébulisation (1) pour un appareil de désinfection de surface par voie aérienne, sensiblement de révolution autour d'un axe (2), comprenant une chambre (3) sensiblement cylindrique d'axe confondu avec l'axe (2) de la buse (1), une canalisation (4) débouchant dans le fond (5) de la chambre (3), préférentiellement au centre dudit fond (5), apte à apporter un produit de traitement, une entrée d'air (6) circonvenant la périphérie de la chambre (3), des ailettes (7) disposées entre l'entrée d'air (6) et la chambre (3) et un diffuseur (8) sensiblement conique s'ouvrant vers l'extérieur, d'axe aligné avec l'axe (2) de la buse (1) et accolé à la chambre (3) du côté opposé au fond *(5), **caractérisée en ce que*** les ailettes (7) sont courbes.

2. La buse (1) selon la revendication **1,** où les ailettes (7) sont parallèles à l'axe (2) de la buse (1) et équi-angulairement réparties autour de l'axe (2) de la buse (1).

3. La buse (1) selon l'une quelconque des revendications **1** ou **2,** où la concavité de la courbure des ailettes (7) est dirigée vers l'intérieur de la buse (1).

4. La buse (1) selon l'une quelconque des revendications **1** à **3,** où les ailettes (7) présentent une forme de spirale initiée sur un cercle (9) centré sur l'axe (2) de la buse (1).

5. La buse (1) selon la revendication **4,** où la spirale est de type logarithmique.

6. La buse (1) selon l'une quelconque des revendications **1** à **5,** où le nombre d'ailettes est compris entre 3 et 10, et préférentiellement égal à 5.

7. La buse (1) selon l'une quelconque des revendications **1** à **6,** où le diffuseur (8) présente un congé (10) arrondi sur son arrête interne de sortie.

8. La buse (1) selon l'une quelconque des revendications **1** à **7,** où le diffuseur (8) comprend, en s'éloignant de la chambre (3), un premier cône (11), un deuxième cône (12) de plus grand diamètre et un décrochement (13) entre les deux cônes (11, 12).

9. La buse (1) selon la revendication **8,** où le décrochement (13) présente un congé arrondi.

10. Un appareil de désinfection de surface par voie aérienne comprenant un réservoir (21) de produit de traitement, un moyen de transport (22), et une canalisation (25) de ce produit jusqu'à une buse (1) et un moto-ventilateur (23) apte à souffler l'air jusqu'à ladite buse (1) **caractérisée en ce que** la buse (1) est telle que définie à l'une 5 quelconque des revendications **1** à 9, et **en ce que** la buse (1) est comprise dans l'appareil de désinfection.

11. Une utilisation d'une buse (1) telle que définie à l'une quelconque des revendications **1** à **9** pour la désinfection de surface par la nébulisation d'un produit de traitement.

## Patentansprüche

1. Zerstäubungsdüse (1) für ein Oberflächendesinfektionsgerät auf Luftbasis, im Wesentlichen rotationssymmetrisch um eine Achse (2), eine im Wesentlichen zylindrische Kammer (3) umfassend, deren Achse mit der Achse (2) der Düse (1) zusammenfällt, eine Leitung (4), die im Boden (5) der Kammer (3) mündet, vorzugsweise im Zentrum des genannten Bodens (5) und geeignet, ein Behandlungsmittel zuzuleiten, einen die Peripherie der Kammer (3) umgebenden Lufteinlass (6), Flügel (7), die zwischen dem Lufteinlass (6) und der Kammer (3) angeordnet sind, und einen im Wesentlichen konischen Diffusor (8), der sich nach außen öffnet und dessen Achse mit der Achse (2) der Düse (1) ausgerichtet ist und der Kammer (3) an der dem Boden (5) gegenüberliegenden Seite angefügt ist, **dadurch gekennzeichnet, dass** die Flügel (7) gekrümmt sind.

2. Düse (1) nach Patentanspruch 1, in der die Flügel (7) zur Achse (2) der Düse (1) parallel verlaufen und um die Achse (2) der Düse (1) in gleichem Winkelabstand verteilt sind.

3. Düse (1) nach irgendeinem der Patentansprüche 1 oder 2, in der die konkave Seite der Flügel (7) dem Inneren der Düse (1) zugewandt ist.

4. Düse (1) nach irgendeinem der Patentansprüche 1 bis 3, in der die Flügel (7) eine Spiralform aufweisen, die auf einem Kreis (9) ihren Ursprung nimmt, der um die Achse (2) der Düse (1) zentriert ist.

5. Düse (1) nach Patentanspruch 4, in der die Spirale logarithmisch ist.

6. Düse (1) nach irgendeinem der Patentansprüche 1 bis 5, in der die Anzahl der Flügel (7) zwischen 3 und 10 liegt und vorzugsweise 5 beträgt.

7. Düse (1) nach irgendeinem der Patentansprüche 1 bis 6, in der der Diffusor (8) eine an seiner inneren Austrittskante abgerundete Ausnehmung (10) aufweist.

8. Düse (1) nach irgendeinem der Patentansprüche 1 bis 7, in der der Diffusor (8) von der Kammer (3) auswärts einen ersten Kegel (11), einen zweiten Kegel (12) größeren Durchmessers und einen Versatz (13) zwischen den beiden Kegeln (11, 12) aufweist.

9. Düse (1) nach Patentanspruch 8, in der der Versatz (13) eine abgerundete Ausnehmung aufweist.

10. Oberflächendesinfektionsgerät auf Luftbasis, einen Vorratsbehälter (21) für Behandlungsmittel umfassend, ein Förderorgan (22) und eine Leitung (25) für dieses Mittel zu einer Düse (1), sowie ein Gebläse (23), geeignet, Luft bis zur genannten Düse (1) zu blasen, **dadurch gekennzeichnet, dass** die Düse (1) der Art ist, wie in irgendeinem der Patentansprüche 1 bis 9 beschrieben, und dadurch, dass die Düse (1) Bestandteil des Desinfektionsgerätes ist.

11. Gebrauch einer Düse (1) nach irgendeinem der Patentansprüche 1 bis 9 zur Oberflächendesinfektion durch Zerstäubung eines Behandlungsmittels.

## Claims

1. A nebulization nozzle (1) for an apparatus for surface disinfection by air, substantially rotating about an axis (2), comprising a substantially cylindrical chamber (3), the axis of which coincides with the axis (2) of the nozzle (1), a pipe (4) opening into the bottom (5) of the chamber (3), preferably in the center of said bottom (5), capable of supplying a treatment product, an air inlet (6) circumventing the periphery of the chamber (3), vanes (7) arranged between the air inlet (6) and the chamber (3), and a substantially conical diffuser (8) opening outwards, the axis of which is aligned with the axis (2) of the nozzle (1) and attached to the chamber (3) on the side opposite the bottom (5), ***characterized in that** the* vanes (7) are curved.

2. The nozzle (1) according to claim 1, wherein the vanes (7) are parallel to the axis (2) of the nozzle (1) and equally angularly distributed around the axis (2) of the nozzle (1).

3. The nozzle (1) according to any one of claims 1 or 2, wherein the concavity of the curvature of the vanes (7) is directed inwardly of the nozzle (1).

4. The nozzle (1) according to any one of claims 1 to 3, wherein the vanes (7) have a spiral shape initiated on a circle (9) centered on the axis (2) of the nozzle (1).

5. The nozzle (1) according to claim 4, wherein the spiral is of the logarithmic type.

6. The nozzle (1) according to any one of claims 1 to 5, wherein the number of vanes is between 3 and 10, and preferably equal to 5.

7. The nozzle (1) according to any one of claims 1 to 6, wherein the diffuser (8) has a rounded fillet (10) on its internal trailing edge.

8. The nozzle (1) according to any one of claims 1 to 7, wherein the diffuser (8) comprises, away from the chamber (3), a first cone (11), a second cone (12) with a larger diameter, and a step (13) between the two cones (11, 12).

9. The nozzle (1) according to claim 8, wherein the step (13) has a rounded fillet.

10. An apparatus for surface disinfection by air comprising a tank (21) of treatment product, a transport means (22), and a pipe (25) for this product to a nozzle (1), and a motorized fan (23) capable of blowing air to said nozzle (1), **characterized in that** the nozzle (1) is as defined in any one of claims 1 to 9, and **in that** the nozzle (1) is comprised in the disinfection apparatus.

11. A use of a nozzle (1) as defined in any one of claims 1 to 9 for surface disinfection by nebulizing a treatment product.
